# EUROPEAN PATENT APPLICATION

(11) **EP 1 310 254 A1**
(43) Date of publication of application: **14.05.2003**
(21) Application number: 01921793.4
(22) Date of filing: 06.04.2001
(51) Int. Cl.: A61K 31/4745, A61K 47/04, A61K 47/26, A61K 9/19, A61P 35/00

(54) **MEDICINAL COMPOSITIONS CONTAINING CAMPTOTHECIN DERIVATIVE AND PH REGULATING AGENT**

(30) Priority: 07.04.2000 JP 2000111919; 01.06.2000 JP 2000169082
(71) Applicant: DAIICHI PHARMACEUTICAL CO., LTD., Chuo-ku, Tokyo 103-0027 (JP)
(72) Inventor: ANDO, Shuichi, Daiichi Pharmaceutical Co., Ltd., Edogawa-ku, Tokyo 134-0081 (JP); SUGIE, S., Daiichi Pharmaceutical Co., Ltd., Takatsuki-shi, Osaka 569-0806 (JP); MORITA, M., Daiichi Pharmaceutical Co., Ltd., Edogawa-ku, Tokyo 134-0081 (JP)
(74) Representative: Kinzebach, Werner, Dr.
(86) International application number: JP0102982
(87) International publication number: WO01076603

(57) **Abstract**

A pharmaceutical preparation which ensures stability and solubility of a hexacyclic camptothecin derivative prepared by adding a ring having a water-soluble group to camptothecin, wherein a pH-adjusting substance and, as occasion demands, a sugar or a sugar alcohol are added to the hexacyclic camptothecin derivative.

## Description

### Technical Field

This invention relates to a pharmaceutical composition containing a camptothecin derivative.

### Background Art

The camptothecin anti-tumor agents so far put on the market were aqueous injections produced by an overkill sterilizing method (treatment at 121°C for 20 minutes). However, in the case of a hexacyclic camptothecin derivative in which a ring having a water-soluble group is added to camptothecin, it has been revealed that the compound is degraded when similar overkill approach is carried out.

The invention provides a pharmaceutical composition which ensures stability and solubility of a hexacyclic camptothecin derivative in which a ring having a water-soluble group is added to camptothecin.

### Disclosure of the Invention

As a result of intensive studies, the present inventors have found that stability of a hexacyclic camptothecin derivative in which a ring having a water-soluble group is added to camptothecin, particularly (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione (to be referred sometimes to as compound A hereinafter), is improved when it is formulated by adding a pH-adjusting substance and, further as occasion demands, a sugar and/or a sugar alcohol.

Accordingly, the invention relates to a pharmaceutical composition which contains a camptothecin derivative and a pH-adjusting substance.

Particularly, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H, 15H) -dione or a salt thereof (it may form a hydrate or the like solvate) and a pH-adjusting substance.

Also, it relates to a pharmaceutical composition which contains (9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof and hydrochloric acid.

Further, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13 (9H,15H) -dione or a salt thereof, a pH-adjusting substance and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose.

Also, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose.

Further, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13 (9H,15H) -dione or a salt thereof, a pH-adjusting substance and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Also, it relates to a pharmaceutical composition which contains (9S)-1-amino-9-ethyl-5-fluoro-2,3-dihydro-9-hydroxy-4-methyl-1H,12H- benzo[de]pyrano[3',4':6,7]indolizino[1,2-b]quinoline-10,13 (9H,15H) -dione or a salt thereof, a pH-adjusting substance and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Further, it relates to apharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Also, it relates to a pharmaceutical composition which contains (9S)-1-amino-9-ethyl-5-fluoro-2,3-dihydro-9-hydroxy-4-methyl-1H, 12H-benzo[de]pyrano[3',4':6,7]indolizino[1,2-b]quinoline-10,13(9H,15H)-dione or a salt thereof, hydrochloric acid and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Further, it relates to a pharmaceutical composition which contains(9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose.

Also, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose, wherein blending amount of maltose is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Further, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose, wherein blending amount of maltose is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Also, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose.

Further, it relates to a pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose, wherein blending amount of maltose is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate.

Also, it relates to a pharmaceutical composition which contains (9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione hydrochloride or methanesulfonate, hydrochloric acid and maltose, wherein blending amount of maltose is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate.

Further, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof and a pH-adjusting substance.

Also, it relates to a pharmaceutical composition which contains (1S,9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof and hydrochloric acid.

Further, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose.

Also, it relates to a pharmaceutical composition which contains (1S,9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof, hydrochloric acid and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose.

Further, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13 (9H,15H) -dione or a salt thereof, apH-adjusting substance and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Also, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13 (9H,15H) -dione or a salt thereof, apH-adjusting substance and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Further, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Also, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and one or more of sugars and/or sugar alcohols selected from the group consisting of maltose, glucose, lactose, saccharose, mannitol, inositol, galactose, ribose, xylose, mannose, sucrose, cellobiose, raffinose and maltotriose, wherein blending amount of the sugar and/or sugar alcohol is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Further, it relates to apharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose.

Also, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose, wherein blending amount of maltose is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Further, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose, wherein blending amount of maltose is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

Also, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13 (9H,15H)-dione hydrochloride or methanesulfonate, hydrochloric acid and maltose.

Further, it relates to a pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose, wherein blending amount of maltose is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate.

Also, it relates to a pharmaceutical composition which contains (1S,9S)-1-amino-9-ethyl-5-fluoro-2,3-dihydro-9-hydroxy-4-methyl-1H, 12H-benzo[de]pyrano[3',4':6,7]indolizino[1,2-b]quinoline-10,13(9H,15H)-dione hydrochloride or methanesulfonate, hydrochloric acid and maltose, wherein blending amount of maltose is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate.

Further, it relates to the aforementioned pharmaceutical composition, wherein pH is a weakly acidic condition.

Also, it relates to the aforementioned pharmaceutical composition, wherein pH is from 3.5 to 5.0.

Further, it relates to the aforementioned pharmaceutical composition, wherein pH is from 4.0 to 4.5.

Also, it relates to a pharmaceutical composition which contains (9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione methanesulfonate, hydrochloric acid and maltose, wherein blending amount of maltose is 25 or 38 parts by weight based on 1 part by weight of (9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione methanesulfonate, and pH is from 4.0 to 4.5.

Further, it relates to a pharmaceutical composition which contains (1S,9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione methanesulfonate, hydrochloric acid and maltose, wherein blending amount of maltose is 25 or 38 parts by weight based on 1 part by weight of (9S) -1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, and pH is from 4.0 to 4.5.

Also, it relates to a freeze-dried preparation which contains the aforementioned pharmaceutical composition.

Further, it relates to an aqueous preparation prepared by dissolving the above freeze-dried preparation.

Also, it relates to a process for producing the abovementioned freeze-dried preparation which comprises adjusting an aqueous solution containing (9S) -1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof to a weakly acidic condition, and then freeze-drying the resulting solution.

Further, it relates to a process for producing the abovementioned freeze-dried preparation which comprises steps of:
(1) preparing an aqueous solution by dissolving sugars and/or sugar alcohols in water;
(2) dissolving (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof;
(3) adjusting pH to a weakly acidic condition with a pH-adjusting substance; and
(4) dispensing the resulting solution into vial after filter-sterilizing it, and followed by freeze-drying it.

Also, it related to a process for producing the abovementioned freeze-dried preparation which comprises steps of:
(1) preparing an aqueous solution by dissolving maltose in water;
(2) dissolving (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate;
(3) adjusting pH to from 3.5 to 5.0 with a pH-adjusting substance; and
(4) dispensing the resulting solution into vial after filter-sterilizing it, and followed by freeze-drying it.

Further, it related to a process for producing the abovementioned freeze-dried preparation which comprises steps of:
(1) preparing an aqueous solution by dissolving maltose in water;
(2) dissolving (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate;
(3) adjusting pH to from 4.0 to 4.5 with hydrochloric acid; and
(4) dispensing the resulting solution into vial after filter-sterilizing it, and followed by freeze-drying it.

Further, use of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof for producing the aforementioned pharmaceutical composition, freeze-dried preparation and aqueous preparation.

Also, use of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate for producing the aforementioned pharmaceutical composition, freeze-dried preparation and aqueous preparation.

Compound A as the camptothecin derivative in the pharmaceutical composition of the invention can be synthesized by the method described in JP-A-5-59061 (the term "JP-A" as used herein means an "unexamined published Japanese patent application"). In this connection, when the camptothecin derivative in the pharmaceutical composition of the invention is described as (9S) -1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or compound A in this description, configuration of the 1-positioned amino group is not particularly limited. When configuration of the 1-positioned amino group of the aforementioned camptothecin derivative is limited in this description, a compound, in which configuration of the 1-positioned amino group is S configuration, is described as (1S,9S)-1-amino-9-ethyl-5-fluoro-2,3-dihydro-9-hydroxy-4-methyl-1H,12H-benzo[de]pyrano[3',4':6,7]indolizino[1,2-b] quinoline-10,13(9H,15H)-dione or compound AS, and a compound, in which configuration of the 1-positioned amino group is R configuration, is described as (1R,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13 (9H,15H) -dione or compound AR. Since both of the compound AS and compound AR have potent anti-tumor effect, they can be used as a mixture of both isomers or the compound AS or compound AR alone as a component of the pharmaceutical composition, but single use of the compound AS is particularly desirable because the compound AS has about two times higher anti-tumor effect than that of the compound AR. The compound A (molecular weight: 435) may form a generally known salt, and examples of the salt include hydrochloride, sulfate, phosphate, tosylate, methanesulfonate and the like. Especially, hydrochloride and methanesulfonate are desirable. In addition, it may form a hydrate or the like solvate. For example, methanesulfonate dihydrate and the like are cited and it can be prepared by the method described in JP-A-8-337584.

The compound A or a salt thereof in the pharmaceutical composition of the invention is used in an amount sufficient for expressing the drug effect, and it is administered at a dose within the range of from about 0.01 mg to about 10 mg, preferably from about 0.1 mg to about 6 mg, based on 1 m² of the body surface area.

Generally, camptothecin derivatives have a lactone ring structure, so it is considered that reduction of the drug effect occurs due to ring-opening of the lactone ring in the structure in the alkaline range. In consequence, it is desirable that a pharmaceutical composition containing a camptothecin derivative having a lactone ring or a salt thereof is maintained within acidic range for the purpose of keeping the lactone ring under closed state. On the other hand, since the compound A or a salt thereof causes salting out under a strongly acidic condition of pH 2.0 or less, its solubility is considerably reduced. From two viewpoints of keeping closed state of lactone ring and ensuring proper solubility of the compound, it is desirable that the pharmaceutical composition of the invention is maintained within a weakly acidic condition by formulating a pH-adjusting substance. In this connection, when the pharmaceutical composition is an aqueous preparation, the term "a weakly acidic condition" as used herein means from about pH 3 to 6, and when the pharmaceutical composition is a freeze-driedpreparation, itmeans that pH of an aqueous solution prepared by dissolving in water is from about pH 3 to 6.

Though the pH-adjusting substance is not particularly limited, with the proviso that it can maintain the pharmaceutical composition of the invention within the weakly acidic range, an acidic substance and/or a basic substance, for example, can be cited. As the acidic substance, hydrochloric acid, acetic acid, sodium acetate, ascorbic acid, sodium ascorbate, phosphoric acid, disodium hydrogenphosphate, sodium dihydrogenphosphate, citric acid, sodium citrate and the like can be exemplified, which may be used alone or as a combination of two or more. Among them, it is desirable to use hydrochloric acid. As the basic substance, sodium hydroxide, glycine, ammonium chloride, triethanolamine and the like can be exemplified, which may be used alone or as a combination of two ormore. Among them, it is desirable to use sodium hydroxide.

The pH-adjusting substance is blended in such an amount that the lactone ring of the camptothecin derivative or a salt thereof is kept under closed state and that 0.7 mg/ml or more, preferably 1 mg/ml or more, of the solubility of the camptothecin derivative or a salt thereof is ensured as a drug solution concentration at the time of the administration of the pharmaceutical composition. Illustratively, it may be such an amount that pH of the pharmaceutical composition can be kept within a weakly acidic condition, preferably an amount which can keep a pH of from 3.5 to 5.0, more preferably an amount which can keep a pH of from 4.0 to 4.5.

Also, it is desirable to further blend the pharmaceutical composition of the invention with a sugar and/or a sugar alcohol for the purpose of improving stability of the camptothecin derivative or a salt thereof. Examples of thereof include maltose, glucose, lactose, saccharose, mannitol, inositol, galactose, ribose, xylose, mannose, sucrose, cellobiose, raffinose, maltotriose and the like, which may be used alone or as a combination of two or more. Among them, it is desirable to use maltose. Blending amount of the sugar and/or sugar alcohol is from 15 to 80 parts by weight, preferably from 25 to 75 parts by weight, more preferably 25 or 38 parts by weight, based on 1 part by weight of the camptothecin derivative or a salt thereof (it may form a hydrate or the like solvate).

As a preparation capable of providing for medicinal use, it is desirable to keep the drug content at 80% by weight or more, preferably 90% by weight or more, under usual storage conditions, at the time of its preparation. Also, it is desirable to keep impurities such as degraded products at a level of 3.5% by weight or less, more preferably 1% by weight or less, at the time of its preparation.

Since the compound A or a salt thereof is considerably unstable against heat and light, changes in appearance, generation of insoluble foreign matter, reduction of the content and the like due, probably, to oxidative degradation of the compound are observed when its severe treatment with heat and light is carried out. In consequence, overkill approach and the like sterilization methods which accompany heating are not suited for the pharmaceutical composition of the invention. In order to obtain a substantially sterilized condition, it is desirable to employ a method in which the pharmaceutical composition is prepared under aseptic condition, a method in which a filter sterilization-treated solution of the pharmaceutical composition is made into a freeze-dried preparation, and the like. Particularly, the method for making a freeze-dried preparation is desirable. In addition, it is desirable for its preservation to use a container having shading ability. For example, brown vials and the like can be cited.

In addition, the pharmaceutical composition of the invention may exist simply as a mixture of a camptothecin derivative or a salt thereof, a pH-adjusting substance and, as occasion demands, a sugar and/or a sugar alcohol, or it may also has an aqueous preparation, freeze-dried preparation or the like known preparation form. Examples of the aqueous preparation include aqueous injections prepared by filter-sterilizing the pharmaceutical composition, another aqueous injections prepared by dissolving the pharmaceutical composition once made into a freeze-dried preparation, and the like. Production method of the freeze-dried preparation is not particularly limited, and any method known by itself may be used.

The following describes the invention further in detail with reference to examples, but the invention is not limited to them. In this connection, the compound AS or a salt thereof used in the tests shown in Examples contains approximately 0.3% by weight of the compound AR or a salt thereof. The compound AR or a salt thereof and degraded products of the compound A or a salt thereof were combined and called "total analogous matter" in the following examples. Also, the concentration of the total analogous matter was indicated as a concentration converted to free form of compound AS.

### Best Mode for Carrying Out the Invention

### (Example 1) Stability of a freeze-dried camptothecin derivative (compound AS hydrochloride)

A 1 mg/ml aqueous solution of compound AS hydrochloride (molecular weight: 472) (a), a 1 mg/ml solution (1:40 by weight) of compound AS hydrochloride containing 4% by weight concentration of mannitol (b), and a 1 mg/ml solution (1:80 by weight) of compound AS hydrochloride containing 8% by weight concentration of maltose (c) were separately freeze-dried and then severe treatment with heat and light was carried out to confirm changes in the content. Colorless vials were used as the container.

As a result, it was revealed that the stability of compound AS is increased by the addition of a sugar or a sugar alcohol, and significant stability was observed particularly when maltose was added (Table 1).

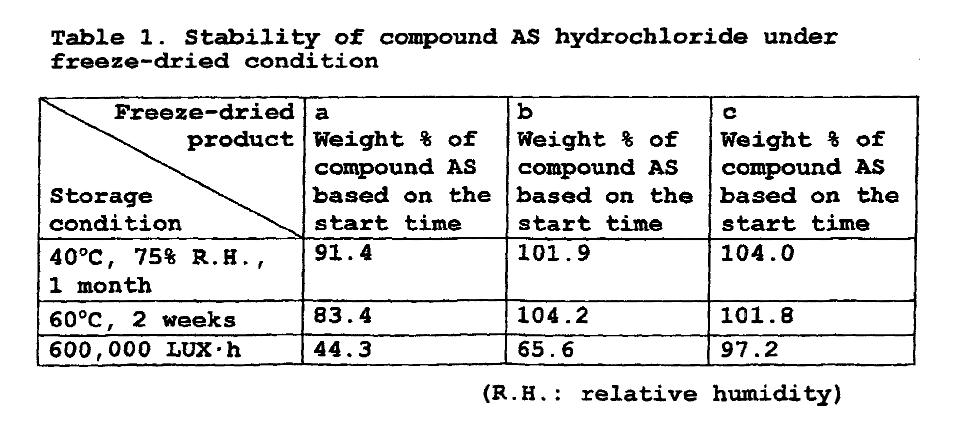

### (Example 2) Stability of a freeze-dried camptothecin derivative (compound AS methanesulfonate)

Mannitol (a), maltose (b) or lactose (c) was added to an aqueous solution (1.5 mg/ml as a concentration converted to free form) of compound AS methanesulfonate (hereinafter, the compound AS methanesulfonate is a dihydrate; molecular weight: 568) to the final concentration of 50 mg/ml (AS methanesulfonate:mannitol=1:25 by weight, AS methanesulfonate:maltose=1:25 by weight and AS methanesulfonate:lactose=1:25 by weight) , and pH was adjusted to about 3.5 by adding hydrochloric acid. Each solution was freeze-dried and then severe treatment with heat and light was carried out to confirm changes in the formation of analogous matter. Colorless vials were used as the container.

As a result, it was revealed that the effect to inhibit analogous matter formation becomes higher when maltose or lactose is added than the case of the addition of mannitol (Table 2) .

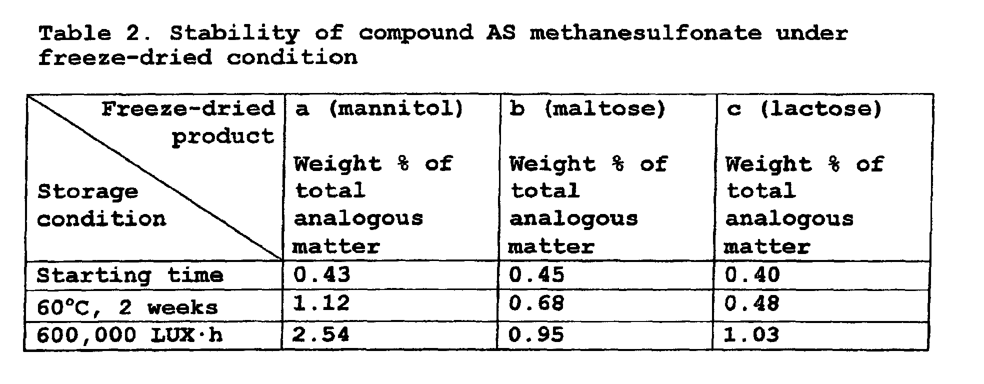

### (Example 3) Stability of a freeze-dried camptothecin derivative (compound AS methanesulfonate) (effect of the addition of acidic substance)

Maltose was added to an aqueous solution (1.0 mg/ml as a concentration converted to free form) of compound AS methanesulfonate to the final concentration of 50 mg/ml (AS methanesulfonate:maltose=1:38 by weight) , and pH was adjusted to 4.0 with hydrochloric acid (a), sodium ascorbate (b) or sodium acetate (c). Each solution was freeze-dried and then severe heat treatment was carried out to confirm changes in the formation of analogous matter. Brown vials were used as the container.

As a result, it was revealed that the effect to inhibit analogous matter formation becomes higher when hydrochloric acid is added than the case of the addition of other acidic substances (Table 3).

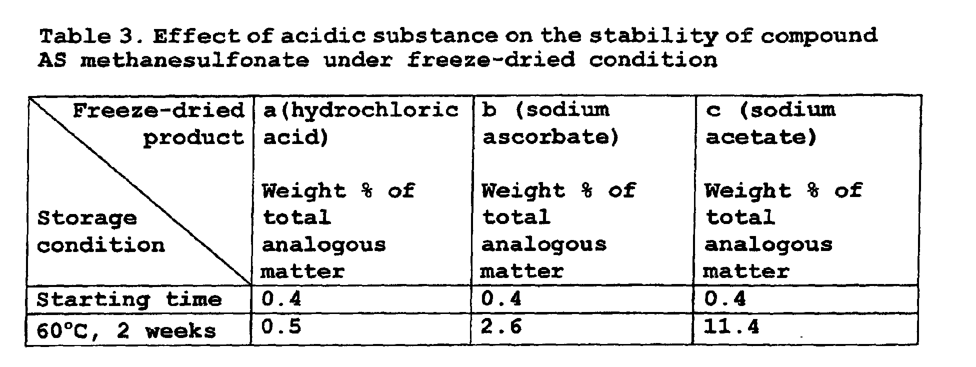

### (Example 4) Stability of a freeze-dried camptothecin derivative (compound AS methanesulfonate) (effect of the addition of maltose)

Maltose was added to an aqueous solution (1.0 mg/ml as a concentration converted to free form) of compound AS methanesulfonate to the final concentration of 50 (AS methanesulfonate:maltose=1:38 by weight) or 100 mg/ml (AS methanesulfonate:maltose=1:77 by weight), and pH was adjusted to 4.0 with hydrochloric acid. Each solution was freeze-dried and then severe heat treatment was carried out to confirm changes in the formation of analogous matter. Brown vials were used as the container.

As a result, it was revealed that difference in the maltose blending ratio exerts influence upon the formation of analogous matter (Table 4).

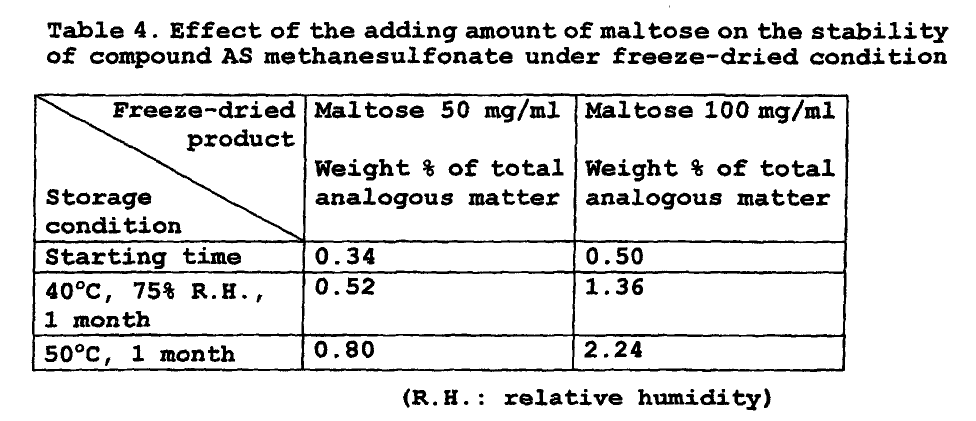

### (Example 5) Stability of a freeze-dried camptothecin derivative (compound AS methanesulfonate) (effect of pH)

Maltose was added to an aqueous solution (1.0 mg/ml as a concentration converted to free form) of compound AS methanesulfonate to the final concentration of 100 mg/ml (AS methanesulfonate:maltose=1:77 by weight) , and pH was adjusted to 3.5, 4.0 or 4.5 by adding hydrochloric acid. Each solution was freeze-dried and then severe heat treatment was carried out to confirm changes in the formation of analogous matter. Brown vials were used as the container.

As a result, it was revealed that the effect to inhibit analogous matter formation becomes most high when pH is adjusted to around 4.5 (Table 5).

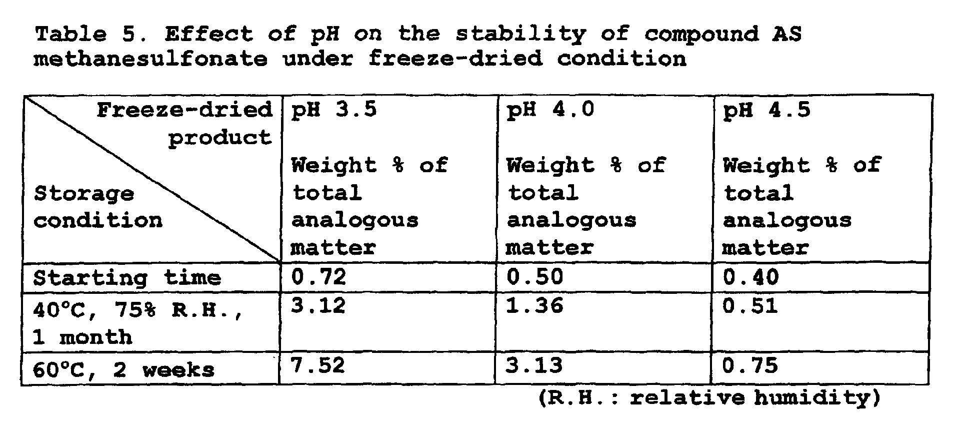

### (Example 6) Stability of a freeze-dried camptothecin derivative (compound AS methanesulfonate) (effect of pH)

Maltose was added to an aqueous solution (1.0 mg/ml as a concentration converted to free form) of compound AS methanesulfonate to the final concentration of 50 mg/ml (AS methanesulfonate:maltose=1:38 by weight) , and pH was adjusted to 2.5, 3.0, 3.5 or 4.0 by adding hydrochloric acid. Each solution was freeze-dried and then severe heat treatment was carried out to confirm changes in the formation of analogous matter. Colorless vials were used as the container.

As a result, it was revealed that the effect to inhibit analogous matter formation becomes most high when pH is adjusted to around 4.0 (Table 6).

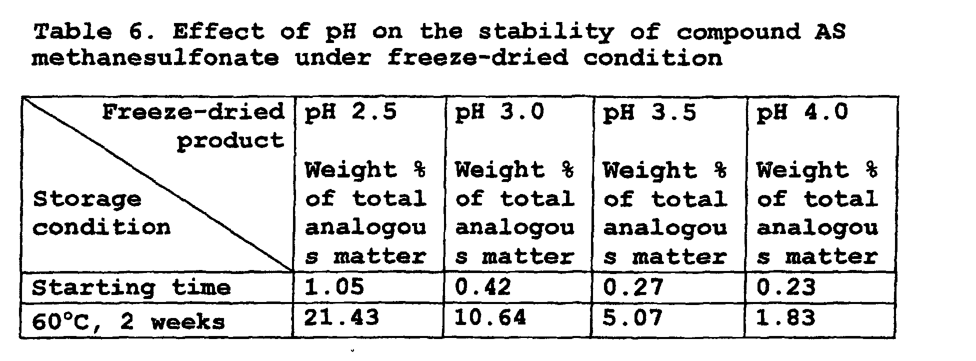

### (Example 7) Changes in pH of a freeze-dried camptothecin derivative (compound AS methanesulfonate) by freeze drying treatment

The pharmaceutical composition containing compound AS methanesulfonate was produced according to the formulation and production scale shown in Table 7 (AS methanesulfonate:maltose=1:19 or 1:38 by weight), end pH of the solution before freeze drying and pH of a solution prepared by dissolving the preparation after freeze drying in water to the same concentration of the solution before freeze drying were measured. As a result, it was revealed that the pH hardly changes by freeze drying treatment.

**Table 7.**

| Changes in pH of compound AS methanesulfonate by freeze drying treatment | | | | |
|---|---|---|---|---|
| Production scale | Formulation | | pH Before freeze drying | pH After freeze drying |
| 28.0 L | Compound AS | 2 mg | 3.67 | 3.83 |
| | Maltose | 50 mg | | |
| | Hydrochloric acid proper amt. Water | total vol. 1 ml | | |
| 28.0 L | Compound AS | 2 mg | 3.73 | 4.00 |
| | Maltose | 50 mg | | |
| | Hydrochloric acid proper amt. Water | total vol. 1 ml | | |
| 90.0 L | Compound AS | 2 mg | 3.85 | 3.97 |
| | Maltose | 100 mg | | |
| | Hydrochloric acid proper amt. Water | total vol. 2 ml | | |

### (Example 8) Stability of a freeze-dried camptothecin derivative (compound AS methanesulfonate) (long term stability)

A 1.4 kg portion of maltose and 73 g (56 g as converted to free form) of compound AS methanesulfonate was dissolved in 22 L of water (AS methanesulfonate:maltose=1:19 by weight) . This solution was adjusted to a pH range of from 3.5 to 3.9 by adding proper amount of hydrochloric acid, filled up to 28 L by adding water, followed by filter-sterilizing, and then dispensed into vials. After freeze drying, this was stored under a condition of 5°C or 25°C with a relative humidity of 60%, and then dissolved in water to the same concentration of the solution before freeze drying to measure pH. Also, changes in the formation of analogous matter were confirmed. Colorless vials were used as the container.

As a result, the pH after freeze drying varied from pH 3.7 to 4.6 including experimental error under each storage condition (Table 8).

Since formation of the total analogous matter until 37 months was 0.33% or less as weight % of the total analogous matter, long term stability of the compound AS methanesulfonate freeze-dried preparation was confirmed (Table 8).

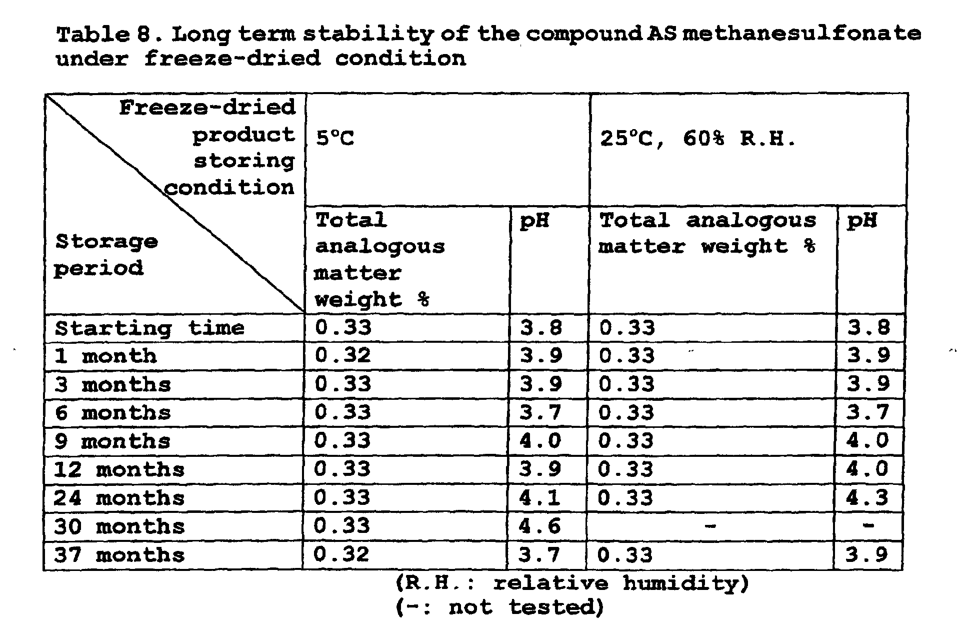

### Industrial Applicability

As described above, the pharmaceutical composition of the invention can be used as a camptothecin derivative preparation having excellent stability and solubility.

## Claims

1. A pharmaceutical composition which contains (9S)-1-amino-9-ethyl-5-fluoro-2,3-dihydro-9-hydroxy-4-methyl-1H,2H-benzo[de]pyrano[3',4':6,7]indolizino[1,2-b]quinoline-10,13(9H,15H)-dione or a salt thereof and a pH-adjusting substance.

2. A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof and a pH-adjusting substance.

3. A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the following group.
maltose, glucose, lactose, saccharose, mannitol, inositol, galactose, ribose, xylose, mannose, sucrose, cellobiose, raffinose and maltotriose.

4. A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the following group.
maltose, glucose, lactose, saccharose, mannitol, inositol, galactose, ribose, xylose, mannose, sucrose, cellobiose, raffinose and maltotriose.

5. A pharmaceutical composition according claim 3 or 4, wherein blending amount of the sugar and/or sugar alcohol is from 15 to 80 parts by weight based on 1 part by weight of (9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof.

6. A pharmaceutical composition according claim 3 or 4, wherein blending amount of the sugar and/or sugar alcohol is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

7. A pharmaceutical composition according any one of claims 1 to 6, wherein said pH-adjusting substance is hydrochloric acid.

8. A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose.

9. A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose.

10. A pharmaceutical composition according claim 8 or 9, wherein blending amount of maltose is from 15 to 80 parts by weight based on 1 part by weight of (9S) -1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof.

11. A pharmaceutical composition according claim 8 or 9, wherein blending amount of maltose is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

12. A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose.

13. A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose.

14. A pharmaceutical composition according claim 12 or 13, wherein blending amount of maltose is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate.

15. A pharmaceutical composition according claim 12 or 13, wherein blending amount of maltose is from 25 to 75 parts by weight based on 1 part by weight of (9S) -1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate.

16. The pharmaceutical composition according to any one of claims 1 to 15, wherein pH is a weakly acidic condition.

17. The pharmaceutical composition according to any one of claims 1 to 15, wherein pH is from 3.5 to 5.0.

18. The pharmaceutical composition according to any one of claims 1 to 15, wherein pH is from 4.0 to 4.5.

19. A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, hydrochloric acid and maltose, wherein blending amount of maltose is 25 or 38 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, and pH is from 4.0 to 4.5.

20. A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, hydrochloric acid and maltose, wherein blending amount of maltose is 25 or 38 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, and pH is from 4.0 to 4.5.

21. A freeze-dried preparation which contains the pharmaceutical composition described in any one of claims 1 to 20.

22. An aqueous preparation prepared by dissolving the freeze-dried preparation described in claim 21.

23. A process for producing a freeze-dried preparation described in claim 21 which comprises adjusting an aqueous
solution containing (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof to a weakly acidic condition, and then freeze-drying the resulting solution.

24. The process according to claim 23 which comprises steps of:
(1) preparing an aqueous solution by dissolving sugars and/or sugar alcohols in water;
(2) dissolving (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof;
(3) adjusting pH to a weakly acidic condition with a pH-adjusting substance; and
(4) dispensing the resulting solution into vial after filter-sterilizing it, and followed by freeze-drying it.

25. The process according to claim 23 or 24 which comprises steps of:
(1) preparing an aqueous solution by dissolving maltose in water;
(2) dissolving (1S,9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione methanesulfonate;
(3) adjusting pH to from 3.5 to 5.0 with a pH-adjusting substance; and
(4) dispensing the resulting solution into vial after filter-sterilizing it, and followed by freeze-drying it.

26. Use of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof for producing the pharmaceutical composition described in any one of claims 1 to 20, the freeze-dried preparation described in claim 21 and the aqueous preparation described in claim 22.

27. Use of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate for producing the pharmaceutical composition described in any one of claims 1 to 20, the freeze-dried preparation described in claim 21 and the aqueous preparation described in claim 22.

## Amended claims

### Amended claims under Art. 19.1 PCT

**1.** A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof and a pH-adjusting substance.

**2.** (Amended) A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the following group.
maltose, glucose, lactose, saccharose, mannitol, inositol, galactose, ribose, xylose, mannose, sucrose, cellobiose, raffinose and maltotriose.

**3.** (Amended) A pharmaceutical composition which contains (9S)-1-amino-9-ethyl-5-fluoro-2,3-dihydro-9-hydroxy-4-methyl-1H,12H-benzo[de]pyrano[3' ,4':6,7]indolizino[1,2-b]quinoline-10,13(9H,15H)-dione or a salt thereof, a pH-adjusting substance and one or more of sugars and/or sugar alcohols selected from the following group, wherein blending amount of the sugar and/or sugar alcohol is from 15 to 80 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.
maltose, glucose, lactose, saccharose, mannitol, inositol, galactose, ribose, xylose, mannose, sucrose, cellobiose, raffinose and maltotriose.

**4.** (Amended) A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the following group, wherein blending amount of the sugar and/or sugar alcohol is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.
maltose, glucose, lactose, saccharose, mannitol, inositol, galactose, ribose, xylose, mannose, sucrose, cellobiose, raffinose and maltotriose.

**5.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof and a pH-adjusting substance.

**6.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the following group.
maltose, glucose, lactose, saccharose, mannitol, inositol, galactose, ribose, xylose, mannose, sucrose, cellobiose, raffinose and maltotriose.

**7.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the following group, wherein blending amount of the sugar and/or sugar alcohol is from 15 to 80 parts by weight based on 1 part by weight of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.
maltose, glucose, lactose, saccharose, mannitol, inositol, galactose, ribose, xylose, mannose, sucrose, cellobiose, raffinose and maltotriose.

**8.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, a pH-adjusting substance and one or more of sugars and/or sugar alcohols selected from the following group, wherein blending amount of the sugar and/or sugar alcohol is from 25 to 75 parts by weight based on 1 part by weight of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.
maltose, glucose, lactose, saccharose, mannitol, inositol, galactose, ribose, xylose, mannose, sucrose, cellobiose, raffinose and maltotriose.

**9.** (Amended) A pharmaceutical composition according any one of claims 1 to 8, wherein said pH-adjusting substance is hydrochloric acid.

**10.** (Amended) A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose.

**11.** (Amended) A pharmaceutical composition which contains (9S)-1-amino-9-ethyl-5-fluoro-2,3-dihydro-9-hydroxy-4-methyl-lH,12H-benzo[de]pyrano[3',4':6,7]indolizino[1,2-b]quinoline-10,13(9H,15H)-dione or a salt thereof, hydrochloric acid and maltose, wherein blending amount of maltose is from 15 to 80 parts by weight based on 1 part by weight of (9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof.

**12.** (Amended) A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose, wherein blending amount of maltose is from 25 to 75 parts by weight based on 1 part by weight of (9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof.

**13.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4'6,7]indolizino[1,2-b]quinoline-10,13(9H,15H)-dione or a salt thereof, hydrochloric acid and maltose.

**14.** (Amended) Apharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof, hydrochloric acid and maltose, wherein blending amount of maltose is from 15 to 80 parts by weight based on 1 part by weight of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

**15.** (Amended) A pharmaceutical composition which contains (1S,9S)-1-amino-9-ethyl-5-fluoro-2,3-dihydro-9-hydroxy-4-methyl-1H,12H-benzo[de]pyrano[3' ,4' :6,7]indolizino[1,2-b]quinoline-10,13(9H,15H)-dione or a salt thereof, hydrochloric acid and maltose, wherein blending amount of maltose is from 25 to 75 parts by weight based on 1 part by weight of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof.

**16.** (Amended) A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose.

**17.** (Amended) A pharmaceutical composition which contains (9S)-1-amino-9-ethyl-5-fluoro-2,3-dihydro-9-hydroxy-4-methyl-1H,12H-benzo[de]pyrano[3',4'6,7]indolizino[1,2-b]quinoline-10,13(9H,15H)-dione hydrochloride or methanesulfonate, hydrochloric acid and maltose, wherein blending amount of maltose is from 15 to 80 parts by weight based on 1 part by weight of (9S) -1-amino-9-ethyl-5-fluoro-2,3-dihydro-9-hydroxy-4-methyl-1H,12H-benzo[de]pyrano[3',4' :6,7]indolizino[1,2-b]quinoline-10,13(9H,15H)-dione hydrochloride or methanesulfonate.

**18.** (Amended) A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose, wherein blending amount of maltose is from 25 to 75 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate.

**19.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose.

**20.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose, wherein blending amount of maltose is from 15 to 80 parts by weight based on 1 part by weight of (1S,9S)-1-amino-9-ethyl-5-fluoro-2,3-dihydro-9-hydroxy-4-methyl-1H,12H- benzo[de]pyrano[3',4':6,7]indolizino[1,2-b]quinoline-10,13(9H,15H)-dione hydrochloride or methanesulfonate.

**21.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione hydrochloride or methanesulfonate, hydrochloric acid and maltose, wherein blending amount of maltose is from 25 to 75 parts by weight based on 1 part by weight of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H) -dione hydrochloride or methanesulfonate.

**22.** (Amended) The pharmaceutical composition according to any one of claims 1 to 21, wherein pH is a weakly acidic condition.

**23.** (Amended) The pharmaceutical composition according to any one of claims 1 to 21, wherein pH is from 3.5 to 5.0.

**24.** (Amended) The pharmaceutical composition according to any one of claims 1 to 21, wherein pH is from 4.0 to 4.5.

**25.** (Amended) A pharmaceutical composition which contains (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, hydrochloric acid and maltose, wherein blending amount of maltose is 25 or 38 parts by weight based on 1 part by weight of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, and pH is from 4.0 to 4.5.

**26.** (Amended) A pharmaceutical composition which contains (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, hydrochloric acid and maltose, wherein blending amount of maltose is 25 or 38 parts by weight based on 1 part by weight of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate, and pH is from 4.0 to 4.5.

**27.** (Amended) A freeze-dried preparation which contains the pharmaceutical composition described in any one of claims 1 to 26.

**28.** (Amended) An aqueous preparation prepared by dissolving the freeze-dried preparation described in claim 27.

**29.** (Amended) A process for producing a freeze-dried preparation described in claim 27 which comprises adjusting an aqueous solution containing (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof to a weakly acidic condition, and then freeze-drying the resulting solution.

**30.** (Amended) A process for producing a freeze-dried preparation described in claim 27 which comprises adjusting an aqueous solution containing (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof to a weakly acidic condition, and then freeze-drying the resulting solution.

**31.** (Amended) The process according to claim 29 or 30 which comprises steps of:
(1) preparing an aqueous solution by dissolving sugars and/or sugar alcohols in water;
(2) dissolving (9S)-1-amino-9-ethyl-5-fluoro-2,3-dihydro-9-hydroxy-4-methyl-1H, 12H-benzo[de]pyrano[3',4':6,7]indolizino[1,2-b]quinoline-10,13(9H,15H)-dione or a salt thereof;
(3) adjusting pH to a weakly acidic condition with a pH-adjusting substance; and
(4) dispensing the resulting solution into vial after filter-sterilizing it, and followed by freeze-drying it.

**32.** (Amended) The process according to claim 29 or 30 which comprises steps of:
(1) preparing an aqueous solution by dissolving sugars and/or sugar alcohols in water;
(2) dissolving (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof;
(3) adjusting pH to a weakly acidic condition with a pH-adjusting substance; and
(4) dispensing the resulting solution into vial after filter-sterilizing it, and followed by freeze-drying it.

**33.** (Amended) The process according to any one of claims 29 to 32 which comprises steps of:
(1) preparing an aqueous solution by dissolving maltose in water;
(2) dissolving (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate;
(3) adjusting pH to from 3.5 to 5.0 with a pH-adjusting substance; and
(4) dispensing the resulting solution into vial after filter-sterilizing it, and followed by freeze-drying it.

**34.** (Amended) Use of (9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione or a salt thereof for producing the pharmaceutical composition described in any one of claims 1 to 26, the freeze-dried preparation described in claim 27 and the aqueous preparation described in claim 28.

**35.** (Amended) Use of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione methanesulfonate for producing the pharmaceutical composition described in any one of claims 1 to 26, the freeze-dried preparation described in claim 27 and the aqueous preparation described in claim 28.
